Europäisches Patentamt

European Patent Office

Office européen des brevets

(11) Veröffentlichungsnummer: **0 277 518**
**A1**

(12) **EUROPÄISCHE PATENTANMELDUNG**

(21) Anmeldenummer: **88100494.9**

(22) Anmeldetag: **15.01.88**

(51) Int. Cl.⁴: **A61M 5/14**

(30) Priorität: **29.01.87 DE 3702610**

(43) Veröffentlichungstag der Anmeldung:
**10.08.88 Patentblatt 88/32**

(84) Benannte Vertragsstaaten:
**AT BE CH DE ES FR GB GR IT LI LU NL SE**

(71) Anmelder: **B. Braun Melsungen AG**
**Carl-Braun Strasse**
**D-3508 Melsungen(DE)**

(72) Erfinder: **Heitmeier, Rolf, Dipl.-Ing.**
**Goethestrasse 8**
**D-3507 Baunatal(DE)**
Erfinder: **Von der Haar, Friedrich, Prof. Dr. rer.**
**nat.**
**Tilsiter Strasse 2**
**D-3508 Melsungen(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Druckinfusionsapparat.**

(57) Druckinfusionsapparate sollen eine hohe Langzeitgenauigkeit der Förderrate haben. Zu diesem Zweck wird die Fließgeschwindigkeit in einer Flüssigkeitsleitung (13) zwischen zwei Sensoren (17 und 18) ermittelt und daraus die Förderrate bestimmt. Eine Markierungsvorrichtung (16) markiert die Flüssigkeit vor den Sensoren (17 und 18) durch selektives Erwärmen oder Injizieren von Fremdstoffen. Die aus der Fließgeschwindigkeit ermittelte Förderrate wird mit einer Soll-Förderrate verglichen und die Differenz steuert die Pumpe (14) so, daß deren Förderrate der Soll-Förderrate entspricht.

## Druckinfusionsapparat

Die Erfindung betrifft einen Druckinfusionsapparat nach dem Oberbegriff der Ansprüche 1 und 2.

Bei der intravenösen Infusionstherapie werden Infusionspumpen mit hoher Infusionsratengenauigkeit benötigt. Ferner ist es wünschenswert, daß der einmal gewählte Volumenstrom über die Dauer einer Langzeitbehandlung von etwa 24 Stunden stabil bleibt.

Peristaltische Infusionspumpen, bei denen ein Schlauch fortlaufend abgequetscht wird, um eine Flüssigkeit zu fördern, liefern keine hinreichend hohe Genauigkeit und Konstanz der Infusionsrate, was u.a. daran liegt, daß der periodisch verformte Schlauch Ermüdungserscheinungen unterliegt. Außerdem ist die Genauigkeit des Volumenstroms von der Justage der mechanischen Pumpenkomponenten und von der Verwendung von Schläuchen mit kalibriertem Schlauchvolumen abhängig.

Bei Verwendung von Kolbenpumpen liegt ein Nachteil darin, daß ein besonderer Aufwand bei der Abdichtung des ansaugenden und des ausstoßenden Kolbens erforderlich ist, weil die Genauigkeit nicht durch Undichtigkeiten beeinträchtigt werden darf.

Bekannt ist eine Vorrichtung zur Durchflußmessung kleiner Flüssigkeitsmengen (DE-OS 33 34 805), bei der ein Flüssigkeitsstrom von einer Pumpe durch ein dünnes rohrförmiges Element getrieben wird. In das rohrförmige Element werden gezielt Gasblasen eingebracht, deren Durchmesser dem Innendurchmesser des rohrförmigen Elements entspricht. Der Flüssigkeitsstrom schiebt die Gasblase vor sich her. An vorgegebenen Meßpunkten wird der Durchlauf der Gasblasen erfaßt. Aus dem Durchlauf wird die Durchflußmenge hergeleitet. Die Laufzeit der Gasblase kann zur Veränderung der Pumpengeschwindigkeit herangezogen werden. Eine derartige Vorrichtung eignet sich in der Regel als Infusionsvorrichtung nicht, weil mit der Flüssigkeit Luft in den Körper des Patienten gelangt, was zu Embolien u.dgl. führen kann.

Der Erfindung liegt die Aufgabe zugrunde, einen Druckinfusionsapparat der im Oberbegriff des Anspruchs 1 angegebenen Art zu schaffen, bei dem die Förderrate mit einfachen Mitteln überwacht wird, ohne daß Luft oder anderes Gas in den Körper des Patienten gelangt.

Die Lösung dieser Aufgabe erfolgt erfindungsgemäß mit den jeweils im kennzeichnenden Teil des Anspruchs 1 und des Anspruchs 2 angegebenen Merkmalen.

Bei dem erfindungsgemäßen Druckinfusionsapparat wird die Strömungsgeschwindigkeit der Flüssigkeit in einem kalibrierten Durchströmvolumen ermittelt und hieraus wird die Förderrate berechnet. Wenn das Meßvolumen und die zum Durchströmen des Meßvolumens erforderliche Zeit bekannt sind, kann die Förderrate durch Verhältnisbildung Meßvolumen:Zeit leicht bestimmt werden. Das Meßvolumen kann entweder aus einem Schlauchabschnitt oder aus einem Rohrabschnitt bestehen. Wichtig ist, daß der Durchmesser dieses Abschnitts konstant ist und keinen zeitlichen Änderungen unterliegt. Da im Bereich des Meßvolumens keine externen Einwirkungen auf die Flüssigkeitsleitung stattfinden, ist die Konstanz des Meßvolumens ohne weiteres erreichbar. Andererseits muß das Meßvolumen auch eine genau definierte Größe haben. Diese Größe kann beispielsweise durch genaue Fertigung des betreffenden Leitungsabschnitts erreicht werden oder (bei relativ hohen Fertigungstoleranzen) durch Messung ermittelt werden. Zweckmäßigerweise besteht der Leitungsabschnitt, der das Meßvolumen enthält, aus einem starren Rohr.

Die Ermittlung der Strömunggeschwindigkeit erfolgt, indem die Flüssigkeit selektiv markiert wird und indem die Zeit gemessen wird, die die Markierung benötigt, um mit der Flüssigkeit von einem Sensor bis zu einem anderen Sensor zu gelangen. Da die Distanz zwischen den Sensoren bekannt ist, kann die Strömungs-oder Fließgeschwindigkeit durch Zeitmessung ermittelt werden. Die Markierung kann entweder durch selektive Temperaturveränderung oder Injizieren von Fremdstoff in die Flüssigkeit erfolgen. Wichtig ist, daß die Sensoren die Markierung erkennen können. Bei Injektion eines Fremdstoffs in die Flüssigkeit sollte zweckmäßigerweise der injizierte Fremdstoff etwa das gleiche spezifische Gewicht haben wie die Flüssigkeit, damit er sich bei vertikaler Anordnung des Meßvolumens innerhalb der Flüssigkeitsströmung nicht wesentlich bewegt. Als Markierungen können ferner kleine Partikel in den Flüssigkeitsstrom injiziert werden, deren Vorhandensein meßtechnisch erfaßt werden kann und die körperverträglich sind bzw. sich innerhalb der Flüssigkeit nach einer gewissen Zeit abbauen oder lösen. Solche Partikel können beispielsweise strahlungsaussendende Teilchen sein. Bei Erzeugung von Markierungen durch selektives Aufheizen wird an oder in der Flüssigkeitsleitung ein Heizelement angebracht, das eine kurzzeitige Aufheizung eines kleinen Flüssigkeitsvolumens ermöglicht. Die Sensoren sprechen auf Temperaturerhöhungen an und erkennen die Anwesenheit des selektiv aufgeheizten Flüssigkeitsvolumens.

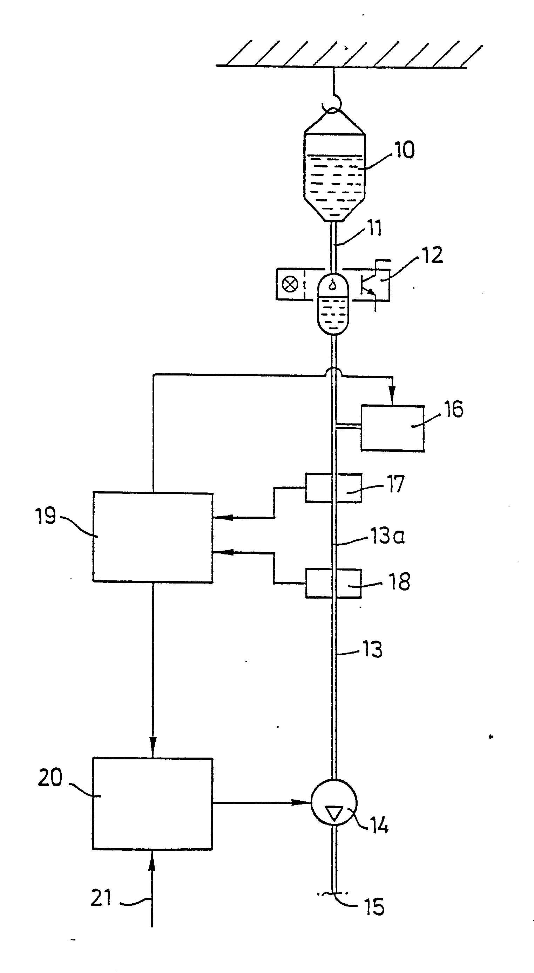

Im folgenden wird unter Bezugnahme auf die einzige Figur der Zeichnung ein Ausführungsbeispiel der Erfindung näher erläutert.

In der Zeichnung ist ein Druckinfusionsapparat nach der Erfindung schematisch dargestellt.

Die Flüssigkeitsquelle 10 besteht aus einem Infusionsbehälter, der eine Infusionslösung enthält und hängend angeordnet ist. Von der Flüssigkeitsquelle 10 führt ein Schlauch 11 über einen opto-elektrischen Tropfenzähler 12 zu einer Flüssigkeitsleitung 13, welche die Pumpe 14 enthält. Der Auslaß 15 der Flüssigkeitsleitung 13 kann an einen Katheter angeschlossen werden, der im Körper des Patienten verlegt ist.

An die Flüssigkeitsleitung 13 ist eine Markierungsvorrichtung 16 angeschlossen. In Strömungsrichtung hinter der Markierungsvorrichtung 16 sind an der Flüssigkeitsleitung 13 zwei Sensoren 17, 18 mit gegenseitigem Abstand hintereinander angeordnet. Der Bereich 13a der Flüssigkeitsleitung 13 zwischen den Sensoren 17 und 18 bildet die Meßstrecke. Dieser Bereich ist zweckmäßigerweise als starres Rohr mit definiertem Querschnitt ausgebildet, so daß sich ein konstantes definiertes Meßvolumen ergibt. Dieses Meßvolumen, das bei dem dargestellten Ausführungsbeispiel im Flüssigkeitsweg vor der Pumpe 14 liegt, könnte auch im Bereich hinter der Pumpe angeordnet sein.

Die Markierungsvorrichtung 16 wird von der Steuerschaltung 19 in festgelegten Intervallen aktiviert, um einen Teil der Flüssigkeit selektiv zu markieren. Im Flüssigkeitsweg hinter der Markierungsstelle ist der erste Sensor 17 angeordnet, der auf die Markierung anspricht, sobald diese den Sensor passiert. In gleicher Weise spricht der zweite Sensor 18 auf die Markierung an, wenn diese den Sensor passiert. Die Sensoren 17 und 18 geben die Erkennungssignale an die Steuerschaltung 19 ab, die die Laufzeit t zwischen den Signalen der Sensoren 17 und 18 mißt. Die Steuerschaltung 19 ermittelt aus dem Meßvolumen V und der Laufzeit t die Förderrate Q nach der Formel

$$Q = \frac{V}{t} \; .$$

Das Meßvolumen V ist

$$V = A \cdot l,$$

worin A die Querschnittsfläche und l die Länge (Sensordistanz) der Meßstrecke 13a ist. Unter der Voraussetzung, daß der Rohrquerschnitt in der Meßstrecke 13a kreisförmig ist, ergibt sich die Ist-Förderrate Q zu

$$Q = \frac{d^2 \cdot l \cdot pi}{4 \cdot t}$$

Hierin ist $d^2$ der Innendurchmesser der Meßstrecke 13.

Die Ermittlung des Volumenstroms Q erfolgt in festen Zeitintervallen. Der Ist-Wert des Volumenstroms wird dem Regler 20 zugeführt, dem außerdem über Leitung 21 ein Sollwert zugeführt wird. Der Regler 20 steuert die Laufgeschwindigkeit der Pumpe 14 so, daß der Ist-Wert der Förderrate die Größe des Sollwertes annimmt.

Da bei der beschriebenen Vorrichtung der Ist-Wert der Förderrate erst eine gewisse Zeit nach dem Einschalten der Pumpe 14 ermittelt werden kann, arbeitet die Regelelektronik in der Zwischenzeit mit einem festen Proportionalitätsfaktor, bis die Regelung einsetzt. Anhand dieses Proportionalitätsfaktors bestimmt sich die Drehzahlvorgabe an die Infusionspumpe, bis ein konkreter Ist-Wert vorliegt.

Die beschriebene Vorrichtung ist unabhängig von der Art der verwendeten Pumpe 14. Als Pumpen können sowohl Schlauchpumpen als auch Kolbenpumpen benutzt werden. Bei tropfengeregelten Pumpen kann auf einfache Weise der geförderte Volumenstrom errechnet werden.

Die beschriebene Vorrichtung liefert eine ausgezeichnete Langzeitstabilität, weil die Meßstrecke 13a keinen mechanischen Verformungen und Spannungen ausgesetzt ist.

## Ansprüche

1. Druckinfusionsapparat mit

einer von einer Flüssigkeitsquelle (10) über eine Pumpe (14) zu einem Auslaß (15) führenden Flüssigkeitsleitung (13),

einer an der Flüssigkeitsleitung (13) vorgesehenen Markierungsvorrichtung (16) zum selektieven Markieren der Flüssigkeit,

mindestens zwei Sensoren (17,18), die hinter der Markierungsvorrichtung (16) mit gegenseitigem Abstand angeordnet sind und auf die Markierung ansprechen,

einer Rechenschaltung (19), die aus der Laufzeit der Markierung zwischen den Sensoren (17,18) die Förderrate berechnet, und

einer Einrichtung zum Verändern der Pumpengeschwindigkeit in Abhängigkeit von der Förderrate

**dadurch gekennzeichnet,**

daß die Markierungsvorrichtung (16) eine Heizvorrichtung aufweist und die Sensoren (17,18) auf Temperaturerhöhungen ansprechen und daß die Einrichtung zum Verändern der Pumpengeschwindigkeit eine Regeleinrichtung (20) ist, die die Pumpengeschwindigkeit entsprechend einer eingestellten Soll-Förderrate regelt.

2. Druckinfusionsapparat mit

einer von einer Flüssigkeitsquelle (10) über eine Pumpe (14) zu einem Auslaß (15) führenden Flüssigkeitsleitung (13),

einer an der Flüssigkeitsleitung (13) vorgesehenen Markierungsvorrichtung (16) zum selektieven Markieren der Flüssigkeit,

mindestens zwei Sensoren (17,18) die hinter der Markierungsvorrichtung (16) mit gegenseitigem Abstand angeordnet sind und auf die Markierung ansprechen,

einer Rechenschaltung (19) die aus der Laufzeit der Markierung zwischen den Sensoren (17,18) die Förderrate berechnet, und

einer Einrichtung zum Verändern der Pumpengeschwindigkeit in Abhängigkeit von der Förderrate

**dadurch gekennzeichnet,**

daß die Markierungsvorrichtung (16) einen Behälter mit Flüssigkeit oder löslichen Partikeln enthält und diese in die Flüssigkeitsleitung (13) injiziert.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-8 605 993 (MEDITEC RESEARCH ASSOCIATES) <br> * Ansprüche 1, 8; Seite 9, Zeilen 28-35; Figuren 1, 5 * <br> --- | 1 | A 61 M 5/14 |
| A | US-A-3 662 598 (SPENCER) <br> * Anspruch 1; Figur 1 * <br> --- | 2 | |
| A | US-A-4 363 327 (CLARK) <br> * Ansprüche 1, 5, 6; Spalte 2, Zeilen 6-12 * <br> --- | 2 | |
| A | DE-A-2 944 979 (SORGATZ) <br> * Zusammenfassung; Ansprüche 1, 2, 6 * <br> --- | 1 | |
| A | EP-A-0 023 457 (OLIVA) <br> * Anspruch 1; Figur 1 * <br> ----- | 1 | |

RECHERCHIERTE SACHGEBIETE (Int. Cl.4)

A 61 M 5/00
G 01 F 1/00

Der vorliegende Recherchenbericht wurde für alle Patentansprüche erstellt

| Recherchenort | Abschlußdatum der Recherche | Prüfer |
|---|---|---|
| BERLIN | 19-04-1988 | PAPA E.R. |